(19) Europäisches Patentamt
European Patent Office
Office européen des brevets

(11) Veröffentlichungsnummer: **0 196 276**
**B1**

(12) # EUROPÄISCHE PATENTSCHRIFT

(45) Veröffentlichungstag der Patentschrift: **18.10.89**

(51) Int. Cl.⁴: **C 07 C 29/40**, C 07 C 31/36, C 07 C 149/36, C 07 C 33/46

(21) Anmeldenummer: **86810135.3**

(22) Anmeldetag: **21.03.86**

(54) **Verfahren zur Herstellung von teilhalogenierten 1-Propanolen.**

(30) Priorität: **28.03.85 CH 1346/85**

(43) Veröffentlichungstag der Anmeldung:
**01.10.86 Patentblatt 86/40**

(45) Bekanntmachung des Hinweises auf die Patenterteilung:
**18.10.89 Patentblatt 89/42**

(84) Benannte Vertragsstaaten:
**CH DE FR GB IT LI NL**

(56) Entgegenhaltungen:
**EP-A- 0 073 569**
**EP-A- 0 157 739**
**DE-A- 1 816 282**
**FR-A- 1 496 633**
**US-A- 3 290 333**

**Houben-Weyl, Methoden der organischen Chemie, Band XIII/2, pp. 689-90, Eugen Müller Ed. Thieme Verlag 1973**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Lang, Robert Werner, Dr., Hagenbachweg 10, CH-4133 Pratteln (CH)**

**EP 0 196 276 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Trifluordichlorpropanolen durch Umsetzung einer organischen Zinkverbindung mit einem Aldehyd.

In der FR-PS 1 496 633 ist die Herstellung von 3,3,3-Trifluor-2,2-dichlor-1-propanol durch die Umsetzung von Formaldehyd, Fluorwasserstoffsäure und 1,1-Dichlor-2,2-difluorethylen im Autoklaven bei erhöhter Temperatur beschrieben. Die Verbindung wird nur in sehr geringen Mengen (weniger als 2%) gebildet. Das 1,1-Dichlor-2,2-difluorethylen ist teuer und zudem muss wegen dessen Flüchtigkeit im Autoklaven gearbeitet werden, was unwirtschaftlich ist.

Gegenstand vorliegender Erfindung ist ein Verfahren zur Herstellung von teilhalogenierten 1-Propanolen der Formel I

$$F_3CCCl_2-\overset{R}{\overset{|}{C}}H-OH \qquad (I),$$

worin R für ein Wasserstoffatom steht oder ein unsubstituierter oder substituierter aliphatischer oder aromatischer Kohlenwasserstoffrest oder ein unsubstituierter oder substituierter aliphatisch- oder aromatisch-heterocyclischer Rest ist, die über ein C-Atom an die CH-Gruppe in Formel I gebunden sind, durch Umsetzung einer Zinkverbindung mit einem Aldehyd, das dadurch gekennzeichnet ist, dass man in einem inerten Lösungsmittel einen Aldehyd der Formel II

$$R-CHO \qquad (II),$$

worin R die zuvor angegebene Bedeutung hat, mit einer Zinkverbindung der Formel III

$$CF_3CCl_2ZnCl\cdot yL \qquad (III),$$

worin y die Zahl 1 oder 2 ist und L ein Lösungsmittelligand aus der Gruppe der N-disubstituierten Säureamide, der N-substituierte Lactame und der organischen Sulfoxide bedeutet, umsetzt, ausgenommen die Umsetzung von $CF_3CCl_3$ mit einem Aldehyd RCHO in Gegenwart von Zink, und danach das 1-Propanol der Formel I isoliert.

R in Formel I enthält als Kohlenwasserstoffrest bevorzugt 1 bis 30, besonders 1 bis 20 und insbesondere 1 bis 12 C-Atome, R als heterocyclischer Rest enthält bevorzugt 1 bis 3 Heteroatome im Ring. Der Ring enthält bevorzugt 4 bis 8, besonders 5 oder 6 Ringglieder. Die Heteroatome sind bevorzugt O, S oder N und es können gleiche oder verschiedene Heteroatome im Ring enthalten sein. Der Rest R ist unsubstituiert oder mit vorzugsweise 1 bis 3 Resten substituiert.

R ist als aliphatischer Kohlenwasserstoffrest bevorzugt unsubstituiertes oder substituiertes Alkyl, Alkenyl, Alkinyl, Cycloalkyl oder Cycloalkenyl, als unsubstituierter oder substituierter aromatischer Kohlenwasserstoffrest Aryl, Aralkenyl, Aralkinyl oder Aralkyl, als unsubstituierter oder substituierter heterocyclischaliphatischer Rest ein 4- bis 6-gliedriger, 1 oder 2 Heteroatome enthaltender heterocyclischer Ring und als aromatisch-heterocyclischer Rest ein 5- oder 6-gliedriger, 1 oder 2 Heteroatome enthaltender heterocyclischer Ring. Das Alkyl, Alkenyl und Alkinyl kann linear oder verzweigt sein. Beispiele für Alkyl sind Methyl, Ethyl, n-Propyl, iso-Propyl, n-, iso- und tertiär-Butyl, Pentyl, Hexyl, Octyl, 2-Ethylhexyl, Nonyl, Decyl, Undecyl, Dodecyl, Tetradecyl, Octadecyl und Eicosyl.

Beispiele für Alkenyl sin Vinyl, Allyl, 1- oder 2-Methylvinyl, Styryl, Butenyl, Pentenyl, Hexenyl, Octenyl, Decenyl und Dodecenyl. Beispiele für Alkinyl sind Ethinyl, Propargyl, Methylethinyl, Phenylethinyl, Butinyl, Pentinyl, Hexinyl, Octinyl, Decinyl und Dodecinyl.

Das Cycloalkyl und Cycloalkenyl kann 3 bis 12, vorzugsweise 3 bis 8 und insbesondere 3 bis 6 Ringkohlenstoffatome enthalten. Beispiele sind Cyclopropyl, Cyclobutyl, Cyclopentyl, Cyclohexyl, Cycloheptyl, Cyclooctyl, Cyclononyl, Cyclodecyl, Cyclododecyl, Cyclopropenyl, Cyclobutenyl, Cyclopentadienyl, Cyclohexenyl, Cyclohexadienyl, Cyclooctenyl, Cyclooctadienyl, Bicyclo-2,2,2-octenyl, Bicyclo-2,2,1-heptenyl.

Der aromatische Kohlenwasserstoffrest kann ein- oder mehrkernig und kondensiert sein. Bevorzugt sind Aryl, Aralkenyl, Aralkinyl oder Aralkyl mit insbesondere 6 bis 18 bzw. 7 bis 18, bzw. 8 bis 18 C-Atomen. Das Aryl ist bevorzugt Phenyl oder Naphthyl. Beispiele sind Phenyl, Naphthyl, Benzyl, Phenylethyl, 2-Phenylpropyl, Naphthylmethyl, Dihydronaphthalin, Indan, Inden, Fluoren, Phenanthren.

R als aliphatisch-heterocyclischer Rest kann 3 bis 7, vorzugsweise 4 bis 6 Ringglieder und je nach Ringgrösse 1 bis 3, bevorzugt 1 oder 2 Heteroatome wie O, S oder N enthalten. Beispiele für Heteroringe, von denen sich der Rest R ableitet, sind: Oxetan, Oxolan, Oxolen, Oxan, Dioxan, Aziridin, Azetidin, Azetin, Pyrrolidin, Pyrrolin, Tetrahydrothiophen, 2,3-Dihydroindol, Dihydrocumaron, Dihydrothionaphthen, Pyrazolidin, Imidazolidin, Oxazolidin, Thiazolidin, Triazolidin, Oxadiazolidin, Morpholin, Piperidin, Tetrahydrochinolin.

R als aromatisch-heterocyclischer Rest enthält bevorzugt 1 bis 3, besonders 1 bis 2 Heteroatome wie O, S und N und stellt bevorzugt einen 5- oder 6-gliedrigen Ring dar. Ferner kommen auch kondensierte Ringsysteme in Frage. Beispiele für Heteroaromaten, von den sich R ableiten kann, sind Pyrrol, Furan, Thiphen, Pyridin, Pyran, Pyrazol, Imidazol, Benzimidazol, Triazin, Oxazol, Thiazol, Pyrimidin, Pyrazin, Chinolin, Chromen, Purin und Xanthen.

Der Rest R kann ein- oder mehrfach mit gleichen oder verschiedenen Resten substituiert sein, bevorzugt ein- bis dreifach. Geeignete Substituenten für R sind zum Beispiel:

$C_1$–$C_{18}$-Alkyl, $C_2$–$C_{18}$-Alkenyl, $C_2$–$C_{18}$-Alkinyl, $C_6$–$C_{18}$-Aryl, $C_7$–$C_{18}$-Aralkyl, $C_7$–$C_{18}$-Alkaryl, $C_8$–$C_{18}$-Alkarylalkyl, $C_1$–$C_{18}$-Alkoxy, $C_1$–$C_{18}$-Alkylthio, $C_6$–$C_{18}$-Aryloxy, $C_7$–$C_{18}$-Aralkoxy, $C_7$–$C_{18}$-Aralkylthio, $C_7$–$C_{18}$-Alkaryloxy, $C_8$–$C_{18}$-Alkaralk-

oxy, $C_7$–$C_{18}$-Aryloxyalkyl, $C_7$–$C_{18}$-Alkoxyaryl, $C_3$–$C_{12}$-Cycloalkyl, $C_3$–$C_{12}$-Cycloalkoxy, $C_3$–$C_{12}$-Cycloalkenyl, $C_3$–$C_{12}$-Cycloalkenyloxy, –COOH, –$CONH_2$, –$COOR^1$, –$CONHR^1$, –$CONR^1R^2$, worin $R^1$ $C_1$–$C_{18}$-Alkyl, Cyclohexyl, Phenyl oder Benzyl ist und $R^2$ unabhängig die gleiche Bedeutung wie $R^1$ hat; –CN, –OH, –SH, –$SO_2H$, –$SO_3H$, –$SO_2R^1$, –$SO_3R^1$, –$PO_2H$, –$PO_3H_2$, –$PO_2R^1$, –$PO_3R^1$, –$NO_2$, –$NH_2$, –$NHR^1$, –$NR^1R^2$, F, Cl, Br, J und –NCO. Die aliphatischen Reste R können durch –CO– oder –C(O)O– unterbrochen sein.

Das Aryl ist bevorzugt Phenyl. Bevorzugt Substituenten sind $C_1$–$C_{12}$-Alkyl, Phenyl, Benzyl, $C_7$–$C_{14}$-Alkylphenyl, $C_8$–$C_{14}$-Alkylbenzyl, $C_1$–$C_{12}$-Alkoxy, Phenoxy, Benzyloxy, $C_7$–$C_{12}$-Alkylphenoxy, $C_8$–$C_{14}$-Alkylbenzyloxy, $C_7$–$C_{12}$-Phenoxyalkyl, $C_7$–$C_{14}$-Alkoxyphenyl, Cyclopentyl, Cyclohexyl, –COOH, –$CONH_2$, –$COOR^1$ mit $R^1$ gleich $C_1$–$C_{12}$-Alkyl; CN, OH, SH, $NH_2$, $NO_2$, F, Cl, Br, wobei die aliphatischen Reste wie zuvor definiert unterbrochen sein können.

Einige Beispiele für Substituenten sind: Methyl, Ethyl, Propyl, Butyl, Vinyl, Allyl, Ethinyl, Propargyl, Phenyl, Benzyl, Methylbenzyl, Methylphenyl, Methoxy, Ethoxy, Propoxy, Butoxy, Methylthio, Phenoxy, Benzyloxy, Methylphenoxy, Methylbenzyloxy, Phenoxymethyl, Methoxyphenyl, Cyclopentyl, Cyclohexyl, Cyclohexenyl, Cyclohexyloxy, Methoxycarbonyl, Methylamino, Dimethylamino, Methylaminocarbonyl, Alkoxyalkyl wie Methoxymethyl oder Methoxyethyl, Alkylaminoalkyl wie Methylaminomethyl oder Dimethylaminoethyl, Phenoxyphenyl, Methoxycarbonylmethyl oder Ethoxycarbonylethyl.

Die Substituenten können ihrerseits substituiert sein. Beispiele für solche Substituenten sind Chlorphenoxy, Fluorbenzyloxy, Hydroxyalkyl wie Hydroxyethyl, Halogenalkyl wie Chlormethyl, Dichlormethyl, Trichlormethyl, Trifluormethyl, Carboxyphenyl, Methoxycarbonylphenyl, Cyanoalkyl wie Cyanoethyl.

In einer bevorzugten Untergruppe ist R $C_1$–$C_4$-Alkyl, $C_5$- oder $C_6$-Cycloalkyl, $C_1$–$C_4$-Halogenalkyl oder unsubstituiertes oder substituiertes Phenyl oder Benzyl. Bevorzugte Substituenten sind Cl, $C_1$–$C_4$-Alkyl, $C_1$–$C_4$-Alkoxy, $C_1$–$C_4$-Alkylthio, Phenylthio- und Benzylthio.

Die Reaktion wird vorzugsweise bei Temperaturen von 0 bis 80 °C, besonders 0 bis 50 °C und insbesondere bei Raumtemperatur durchgeführt, wobei die Reaktionszeiten von Stunden bis Tage betragen können. Wird die Reaktion unter Einwirkung von Ultraschall z.B. im Bereich von 22–38 kHz vorgenommen, so lassen sich die Reaktionszeiten erheblich reduzieren. Es ist zweckmässig, die Reaktion unter Schutzgasatmosphäre durchzuführen, z.B. Stickstoff oder Edelgasen, und auf Luft- und Feuchtigkeitsauschluss zu achten.

Die Aldehyde der Formel II sind bekannt, kommerziell erhältlich oder sie können nach bekannten Verfahren hergestellt werden. Die Reaktanden werden vorteilhaft in äquimolaren Mengen eingesetzt, wobei es manchmal zweckmässig sein kann, einen geringen Überschuss der Aldehyde der Formel II zu verwenden.

Die Zinkverbindungen der Formel III sind neu. In Formel III stellt L in seiner Bedeutung als N-disubstituiertes Säureamid bevorzugt ein Carbonsäureamid dar, das insbesondere der Formel

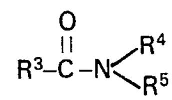

entspricht, worin $R^3$ für ein Wasserstoffatom, unsubstituiertes oder mit Halogen, besonders Fluor oder Chlor, substituiertes Alkyl mit 1 bis 12, besonders 1 bis 4 C-Atomen, unsubstituiertes oder mit $C_1$–$C_4$-Alkyl oder Halogen substituiertes Cycloalkyl mit 4 bis 7 Ringkohlenstoffatomen, Alkenyl mit 2 bis 12, besonders 2 bis 4 C-Atomen, Phenyl, Benzyl oder –$NR^4R^5$ steht, und $R^4$ und $R^5$ unabhängig voneinander $C_1$–$C_{12}$-Alkyl, Cycloalkyl mit 5 oder 6 Ringkohlenstoffatomen, oder $R^4$ und $R^5$ zusammen gegebenenfalls durch –O–, –S– oder –$NR^6$– ($R^6$ gleich $C_1$–$C_4$-Alkyl) unterbrochenes Tetra- oder Pentamethylen bedeuten.

$R^3$ steht besonders bevorzugt für ein Wasserstoffatom oder Methyl. $R^4$ und $R^5$ bedeuten besonders Methyl oder Ethyl.

Beispiele für Säureamide sind Dimethylformamid, Diethylformamid, Dimethylacetamid, Tetramethylharnstoff und N-Formylpyrrolidin. Besonders bevorzugt ist Dimethylformamid.

L in seiner Bedeutung als N-Substituiertes Lactam entspricht bevorzugt der Formel

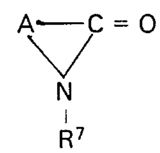

worin A für Di-, Tri-, Tetra- oder Pentamethylen steht, und $R^7$ $C_1$–$C_{12}$, besonders $C_1$–$C_4$-Alkyl, Cyclohexyl oder Cyclopentyl bedeutet. $R^7$ ist besonders Methyl oder Ethyl. Beispiele für solche Lactame sind N-Methylpropiolactam, N-Methylpyrrolidon, N-Ethylpyrrolidon, N-Methylpiperidinon, N-Methyl-ε-caprolactam. Bevorzugt ist N-Methylpyrrolidon.

L in seiner Bedeutung als organisches Sulfoxid entspricht bevorzugt der Formel

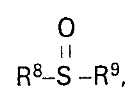

,

worin $R^8$ und $R^9$ unabhängig voneinander $C_1$–$C_{12}$–, besonders $C_1$–$C_4$-Alkyl oder $R^8$ und $R^9$ zusammen Tetra- oder Pentamethylen bedeuten. Beispiele sind Dimethyl-, Methylethyl- und Diethylsulfoxid, Tetramethylensulfoxid und Pentamethylensulfoxid.

Von den Zinkverbindungen der Formel III werden jene bevorzugt verwendet, in denen L ein N-disubstituiertes Carbonsäureamid ist. In Formel III ist y bevorzugt 2. Eine besonders bevorzugt

verwendete Zinkverbindung ist $CF_3CCl_2ZnCl\cdot 2$ Dimethylformamid.

Die Herstellung der Zinkverbindungen der Formel III erfolgt in an sich bekannter Weise z.B. durch die direkte Umsetzung von Zink, das zweckmässig als Zinkstaub vorliegt, mit 1,1,1-Trifluor-2,2,2-trichlorethan unter Luft- und Feuchtigkeitsausschluss und in einem inerten organischen Lösungsmittel in Gegenwart des Lösungsmittelliganden. Bevorzugt entspricht das Lösungsmittel dem Lösungsmittelliganden L. Vorteilhaft wird die Reaktionsmischung gekühlt. Die erfindungsgemässen Zinkverbindungen können dann in üblicher Weise durch Entfernen des Lösungsmittel oder Kristallisation isoliert werden.

In einem anderen Herstellverfahren kann man so vorgehen, dass man bekannte Etheraddukte von $CF_3CCl_2ZnCl$ (vgl. US-PS 3 290 333) in einem Lösungsmittel L löst und gegebenenfalls erwärmt bis etwa 100 °C, wobei sich durch Ligandenaustausch die erfindungsgemäss zu verwendenden Addukte bilden, die dann in bekannter Weise isoliert werden können.

Bei den Zinkverbindungen der Formel III handelt es sich um kristalline Verbindungen, die unter Luft- und Feuchtigkeitsauschluss stabil sind.

Geeignete inerte organische Lösungsmittel sind bevorzugt polare, besonders polare, aprotische Lösungsmittel, die alleine oder in Mischungen aus mindestens zwei Lösungsmitteln verwendet werden können. Geeignete Lösungsmittel sind zum Beispiel Ether wie Diethylether, Dibutylether, Tetrahydrofuran, Dioxan, Dimethylethylenglykol-, Dimethyldiethylenglykol-, Diethyldiethylenglykol-, Dibutyldiethylenglykol-, Dimethyltriethylenglykolether, Carbonsäureester und Lactone wie Propylencarbonat, Essigsäureethylester, Propionsäuremethylester, Benzoesäureethylester, Ethylglykoldiacetat, 2-Methoxyethylacetat, γ-Butyrolacton, γ-Valerolacton und Mevalolacton, Sulfoxide, wie Dimethylsulfoxid, Tetramethylensulfoxid, Sulfone wie Dimethylsulfon, Diethylsulfon, Trimethylensulfon, Tetramethylensulfon, und besonders N-disubstituierte Säureamide wie z.B. Dimethylformamid, Dimethylacetamid, N-Methylpyrrolidon und Hexamethylphosphorsäuretriamid. Besonders bevorzugt ist Dimethylformamid.

In einer bevorzugten Ausführungsform entspricht das inerte Lösungsmittel dem Lösungsmittelliganden L.

Es hat sich als zweckmässig erwiesen, die Zinkverbindung der Formel III in einer ersten Reaktionsstufe in situ herzustellen, z.B. durch Reaktion von Zinkmetall mit $CF_3$–$CCl_3$ in einem Lösungsmittel, das dem Lösungsmittelliganden L entspricht. Es ist aber auch möglich, eine Zinkverbindung der Formel III zunächst in einem inerten Lösungsmittel zu lösen.

Zur Isolierung der Verbindungen der Formel I kann das Reaktionsgemisch zunächst mit einer Säure, bevorzugt verdünnten wässrigen Säuren wie Salzsäure oder Schwefelsäure behandelt werden. Hierbei kann man so vorgehen, dass man das Reaktionsgemisch in die verdünnte Säure giesst, wobei die Säure mit Eis vermischt sein kann. Hierauf gibt man ein Lösungsmittel wie z.B. Ether zu und mischt die wässrige und organische Phase zum Herauslösen der Verbindungen. Nach dem Abtrennen der organischen Phase kann die rohe Verbindung durch Verdampfen des Lösungsmittels erhalten werden. Das Rohprodukt kann in üblicher Weise durch Kristallisation, Destillation oder chromatographische Methoden gereinigt werden.

Mit dem erfindungsgemässen Verfahren werden die Verbindungen der Formel I in hoher Ausbeute und Reinheit unter milden Reaktionsbedingungen in einfachen technischen Apparaturen erhalten. Vorteilhaft ist besonders, dass kein metallisches Zink gleichzeitig mit einem Aldehyd im Reaktionsgemisch vorhanden ist, wodurch Nebenreaktionen wie z.B. die Reduktion von Substituenten im Aldehyd vermieden werden.

Die Verbindungen der Formel I sind wertvolle Zwischenprodukte zur Herstellung von Wirkstoffen mit $CF_3$-Gruppen im Bereich der Pharmazeutika und der Agrochemikalien. Es wurde ferner gefunden, dass sich die Alkohole der Formel I und besonders deren Chlorameisensäureester hervorragend als O- oder N-Schutzgruppen insbesondere in der Peptidsynthese eignen (vgl. EP-A-0 157 739). Diese Schutzgruppen sind unter milden Reaktionsbedingungen abspaltbar. Ferner weisen sie eine erhöhte Lipophilie auf, die zudem durch die Wahl des Restes R in Formel I in einem weiten Bereich variiert werden kann. Durch die erhöhte Lipophilie werden Reaktionen auch in weniger polaren Lösungsmitteln möglich und ferner kann eine spezifische Anpassung der Schutzgruppe an die jeweiligen Reaktionsbedingungen erfolgen.

Die nachfolgenden Beispiele erläutern die Erfindung näher.

Beispiele 1–3

a) In einem 1 Liter Dreihalsrundkolben werden 65,4 g (1 mol) Zinkstaub (aktiviert nach Fieser & Fieser) in 500 ml DMF suspendiert und bei Raumtemperatur langsam mit 188 g (≙ 120 ml; 1 mol) frisch über einem Molekularsieb getrocknet und anschliessend destilliertem $CF_3CCl_3$ versetzt. Nach wenigen Minuten beginnt das Zink in Lösung zu gehen, wobei sich die Reaktionsmischung erwärmt. Die Reaktionstemperatur wird mit externer Kühlung unter 30 °C gehalten. Abschliessend wird 1 Stunde nachgerührt und unter Schutzgas über Filterflockenmasse «Selecta» (Fa. Schleicher & Schüll) filtriert. In das rotbraune, klare Filtrat wird unter kräftigem Rühren 1 mol Aldehyd (siehe nachfolgende Tabelle) zugegeben und bis zum vollständigen Umsatz ausrühren gelassen. Anschliessend wird die Lösung auf eine Mischung aus 500 ml 10%iger wässriger HCl/300 g Eis gegossen und 5× mit je 300 ml Ethylether extrahiert. Es wird mit 200 ml 2%iger wässriger HCl nachgewaschen, über $MgSO_4$ getrocknet und am Rotationsverdampfer eingeengt. Der Rückstand wird je nachdem destilliert (Beispiel 1), umkristallisiert (Beispiel 3) bzw. chromatographiert (Beispiel 2). Die Ausbeuten betragen 70% (Beispiel 1), 82% (Beispiel 2) und 91% (Beispiel 3).

b) In einem 1 Liter Dreihalsrundkolben werden unter Schutzgas und externer Kühlung 1 mol $CF_3CCl_2ZnCl\cdot$Diethylether mit 500 ml DMF versetzt. Nach Erreichen von Raumtemperatur wird unter kräftigem Rühren 1 mol Aldehyd (siehe nachfolgende Tabelle) zugegeben und bis zum vollständigen Umsatz ausrühren gelassen. Die Aufarbeitung erfolgt gemäss a). Die Ausbeuten betragen 70% (Beispiel 1), 78% (Beispiel 2) und 89% (Beispiel 3).

Aldehyd: R-CHO; Propanol der Formel I:

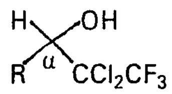

| Beispiel Nr. | R | δHα[a)] | Smp./Sdp. (°C) |
|---|---|---|---|
| 1 | $CCl_3$ | 4,80 ppm | 85°/26 mbar |
| 2 | o-$C_6H_5CH_2SC_6H_4$ | 5,82 ppm | gelbes Öl |
| 3 | 2,6-Dimethoxyphenyl | 5,92 ppm | 75° (farblose Kristalle) |

a) $^1$H-NMR ($CDCl_3$) δ rel. zur TMS = 0 ppm

## Patentansprüche

1. Verfahren zur Herstellung von teilhalogenierten 1-Propanolen der Formel I

$$F_3CCCl_2-\underset{}{\overset{R}{C}}H-OH \qquad (I),$$

worin R für ein Wasserstoffatom steht oder ein unsubstituierter oder substituierter aliphatischer oder aromatischer Kohlenwasserstoffrest oder ein unsubstituierter oder substituierter aliphatisch- oder aromatisch-heterocyclischer Rest ist, die über ein C-Atom an die CH-Gruppe in Formel I gebunden sind, durch Umsetzung einer Zinkverbindung mit einem Aldehyd, dadurch gekennzeichnet, dass man in einem inerten Lösungsmittel einen Aldehyd der Formel II

$$R-CHO \qquad (II),$$

worin R die zuvor angegebene Bedeutung hat, mit einer Zinkverbindung der Formel III

$$CF_3CCl_2ZnCl\cdot yL \qquad (III),$$

worin y die Zahl 1 oder 2 ist und L ein Lösungsmittelligand aus der Gruppe der N-disubstituierten Säureamide, der N-substituierten Lactame und der organischen Sulfoxide bedeutet, umsetzt, ausgenommen die Umsetzung von $CF_3CCl_3$ mit einem Aldehyd RCHO in Gegenwart von Zink, und danach das 1-Propanol der Formel I isoliert.

2. Verfahren gemäss Anspruch 1, dadurch gekennzeichnet, dass die Reaktion bei Raumtemperatur durchgeführt wird.

3. Verfahren gemäss Anspruch 1, dadurch gekennzeichnet, dass die Umsetzung unter Einwirkung von Ultraschall vorgenommen wird.

4. Verfahren gemäss Anspruch 1, dadurch gekennzeichnet, dass das Lösungsmittel ein polares aprotisches Lösungsmittel ist.

5. Verfahren gemäss Anspruch 1, dadurch gekennzeichnet, dass das Lösungsmittel ein N-disubstituiertes Säureamid ist.

6. Verfahren gemäss Anspruch 1, dadurch gekennzeichnet, dass man zur Isolierung der 1-Propanole der Formel I das Reaktionsgemisch mit einer verdünnten wässrigen Säure behandelt.

7. Verfahren gemäss Anspruch 1, dadurch gekennzeichnet, dass das inerte Lösungsmittel dem Lösungsmittelliganden L in Formel III entspricht.

8. Verfahren gemäss Anspruch 1, dadurch gekennzeichnet, dass der Aldehyd der Formel II und die Zinkverbindung der Formel III in äquimolaren Mengen eingesetzt werden.

9. Verfahren gemäss Anspruch 1, dadurch gekennzeichnet, dass die Zinkverbindung der Formel III in einer ersten Reaktionsstufe in situ hergestellt und dann mit einem Aldehyd der Formel II umgesetzt wird.

10. Verfahren gemäss Anspruch 1, dadurch gekennzeichnet, dass man eine Zinkverbindung der Formel III in einem inerten Lösungsmittel löst und dann mit einem Aldehyd der Formel II umsetzt.

11. Verfahren gemäss Anspruch 1, dadurch gekennzeichnet, dass es unter einem inerten Schutzgas durchgeführt wird.

12. Verfahren gemäss Anspruch 1, dadurch gekennzeichnet, dass L in Formel III ein N-disubstituiertes Carbonsäureamid ist.

13. Verfahren gemäss Anspruch 1, dadurch gekennzeichnet, dass y in Formel III für 2 steht.

14. Verfahren gemäss Anspruch 1, dadurch gekennzeichnet, dass die Zinkverbindung der Formel III $CF_3CCl_2ZnCl\cdot 2$ Dimethylformamid ist.

15. Verfahren gemäss Anspruch 1, dadurch gekennzeichnet, dass R in Formel II als Kohlenwasserstoffrest 1 bis 30 C-Atome und als heterocyclisch der Rest 1 bis 3 Heteroatome im Ring enthält.

16. Verfahren gemäss Anspruch 1, dadurch gekennzeichnet, dass Substituenten für R in Formel II $C_1$–$C_{18}$-Alkyl, $C_2$–$C_{18}$-Alkenyl, $C_2$–$C_{18}$-Alkinyl, $C_6$–$C_{18}$-Aryl, $C_7$–$C_{18}$-Aralkyl, $C_7$–$C_{18}$-Alkaryl, $C_8$–$C_{18}$-Alkarylalkyl, $C_1$–$C_{18}$-Alkoxy, $C_1$–$C_{18}$-Alkylthio, $C_6$–$C_{18}$-Aryloxy, $C_7$–$C_{18}$-Aralkoxy, $C_7$–$C_{18}$-Alkaryloxy, $C_8$–$C_{18}$-Alkaralkoxy, $C_7$–$C_{18}$-Aryloxyalkyl, $C_7$–$C_{18}$- Alkoxyaryl, $C_3$-$C_{12}$-Cycloalkyl, $C_3$-$C_{12}$-Cycloalkoxy, $C_3$-$C_{12}$-Cycloalkenyl, $C_3$–$C_{12}$-Cycloalkenyloxy, $-COOH$, $-CONH_2$, $-COOR^1$, $-CONHR^1$, $-CONR^1R^2$, worin $R^1$ $C_1$–$C_{18}$-Alkyl, Cyclohexyl, Phenyl oder Benzyl ist und $R^2$ unabhängig die gleiche Bedeutung wie $R^1$ hat; $-CN$, $-OH$, $-SH$, $-SO_2H$, $-SO_3H$, $-SO_2R^1$,

$-SO_3R^1$, $-PO_2H$, $-PO_3H_2$, $-PO_2R^1$, $-PO_3R^1$, $-NO_2$, $-NH_2$, $-NHR^1$, $-NR^1R^2$, F, Cl, Br, J und –NCO, ist, wobei die aliphatischen Reste R durch –CO– oder –C(O)O– unterbrochen sein können.

17. Verfahren gemäss Anspruch 1, dadurch gekennzeichnet, dass R in Formel II 1- bis 3-fach substituiert ist.

18. Verfahren gemäss Anspruch 1, dadurch gekennzeichnet, dass R in Formel II als aliphatischer Kohlenwasserstoffrest unsubstituiertes oder substituiertes Alkyl, Alkenyl, Alkinyl, Cycloalkyl oder Cycloalkenyl, als unsubstituierter oder substituierter aromatischer Kohlenwasserstoffrest Aryl, Aralkenyl, Aralkinyl oder Aralkyl, als unsubstituierter oder substituierter heterocyclisch-aliphatischer Rest ein 4- bis 6-gliedriger, 1 oder 2 Heteroatome enthaltender heterocyclischer Ring und als aromatisch-heterocyclischer Rest ein 5- oder 6-gliedriger, 1 oder 2 Heteroatome enthaltender heterocyclischer Ring ist.

## Revendications

1. Procédé de préparation de propanols-1 partiellement halogénés de formule I ci-dessous:

$$F_3CCCl_2-\overset{\displaystyle R}{\overset{|}{C}}H-OH \qquad (I),$$

(dans laquelle R désigne un atome d'hydrogène ou un radical hydrocarboné aliphatique ou aromatique avec ou sans substituants ou encore un radical aliphatique- ou aromatique-hétérocyclique avec ou sans substituants, lié par un atome de carbone au groupe CH de la formule I) par réaction d'un composé de zinc avec un aldéhyde, procédé caractérisé en ce que l'on fait réagir dans un solvant inerte un aldéhyde de formule II:

$$R-CHO \qquad (II)$$

avec un composé de zinc de formule III:

$$CF_3CCl_2ZnCl \cdot yL \qquad (III)$$

(y étant le nombre 1 ou 2 et L désignant un coordinat (ligand) solvant pris parmi des amides disubstitués sur l'azote, des lactames substitués sur l'azote et des sulfoxydes organiques), à l'exclusion de la réaction de $CF_3CCl_3$ avec un aldéhyde RCHO en présence de zinc, puis on isole le propanol-1 de formule I formé.

2. Procédé selon la revendication 1, caractérisé en ce que la réaction est effectuée à la température ambiante.

3. Procédé selon la revendication 1, caractérisé en ce que la réaction est effectuée en présence d'ultrasons.

4. Procédé selon la revendication 1, caractérisé en ce que le solvant est un solvant polaire aprotique.

5. Procédé selon la revendication 1, caractérisé en ce que le solvant est un amide disubstitué à l'azote.

6. Procédé selon la revendication 1, caractérisé en ce que pour isoler le propanol-1 de formule I formé on traite le mélange de réaction avec un acide aqueux dilué.

7. Procédé selon la revendication 1, caractérisé en ce que le solvant inerte correspond au coordinat solvant L de la formule III.

8. Procédé selon la revendication 1, caractérisé en ce que l'aldéhyde de formule II et le composé de zinc de formule III sont employés dans des proportions équimolaires.

9. Procédé selon la revendication 1, caractérisé en ce que l'on forme le composé de zinc de formule III in situ dans une première étape réactionnelle pour le faire réagir avec l'aldéhyde de formule II.

10. Procédé selon la revendication 1, caractérisé en ce que l'on dissout le composé de zinc de formule III dans un solvant inerte avant de le faire réagir avec l'aldéhyde de formule II.

11. Procédé selon la revendication 1, caractérisé en ce qu'il est exécuté sous une atmosphère de gaz protecteur inerte.

12. Procédé selon la revendication 1, caractérisé en ce que L, dans la formule III, est un carboxamide disubstitué à l'azote.

13. Procédé selon la revendication 1, caractérisé en ce que y, dans la formule III, est le nombre 2.

14. Procédé selon la revendication 1, caractérisé en ce que le composé de zinc de formule III est le composé de $CF_3CCl_2ZnCl$ avec 2 mol de diméthylformamide.

15. Procédé selon la revendication 1, caractérisé en ce que R, dans la formule II, a de 1 à 30 atomes de carbone comme radical hydrocarboné, et contient de 1 à 3 hétéroatomes comme radical hétérocyclique.

16. Procédé selon la revendication 1, caractérisé en ce que les substituants de R dans la formule II sont les suivants: $C_1$–$C_{18}$-alkyles, $C_2$–$C_{18}$-alcényles, $C_2$–$C_{18}$-alcynyles, $C_6$–$C_{18}$-aryles, $C_7$–$C_{18}$-aralkyles, $C_7$–$C_{18}$-alcaryles, $C_8$–$C_{18}$-alcarylalkyles, $C_1$–$C_{18}$-alcoxy, $C_1$–$C_{18}$-alkylthio, $C_6$–$C_{18}$-aryloxy, $C_7$–$C_{18}$-aralcoxy, $C_7$–$C_{18}$-alcaryloxy, $C_8$–$C_{18}$-alcaralcoxy, $C_7$–$C_{18}$-aryloxyalkyles, $C_7$–$C_{18}$-alcoxyaryles, $C_3$–$C_{12}$-cycloalkyles, $C_3$–$C_{12}$-cycloalcoxy, $C_3$–$C_{12}$-cycloalcényles, $C_3$–$C_{12}$-cycloalcényloxy, –COOH, $-CONH_2$, $-COOR^1$, $-CONHR^1$, $-CONR^1R^2$, $R^1$ étant un alkyle en $C_1$–$C_{18}$, un cyclohexyle, un phényle ou un benzyle et $R^2$, indépendamment étant l'un des groupes indiqués pour $R^1$; –CN, –OH, –SH, $-SO_2H$, $-SO_3H$, $-SO_2R^1$, $-SO_3R^1$, $-PO_2H$, $-PO_3H_2$, $-PO_2R^1$, $-PO_3R^1$, $-NO_2$, $-NH_2$, $-NHR^1$, $-NR^1R^2$, F, Cl, Br, I et –NCO, les radicaux aliphatiques R pouvant être interrompus par les groupes –CO– ou –C(O)O–.

17. Procédé selon la revendication 1, caractérisé en ce que R, dans la formule II, a de 1 à 3 substituants.

18. Procédé selon la revendication 1, caractérisé en ce que R, dans la formule II, en tant que radical hydrocarboné aliphatique, est un alkyle, un alcényle, un alcynyle, un cycloalkyle ou un cycloalcényle avec ou sans substituants, en tant que radical hydrocarboné aromatique substitué

ou non, c'est un aryle, un aralcényle, un aralcynyle ou un aralkyle, entant que radical hétérocyclo-aliphatique substitué ou non, l'hétérocycle a de 4 à 6 chaînons et un ou deux hétéroatomes, et en tant que radical aromatico-hétérocyclique, l'hétérocycle a 5 ou 6 chaînons et un ou deux hétéroatomes.

**Claims**

1. A process for the preparation of a partially halogenated 1-propanol of formula I

$$F_3CCCl_2-\overset{\displaystyle R}{\underset{}{C}}H-OH \qquad (I),$$

in which R is a hydrogen atom or an unsubstituted or substituted aliphatic or aromatic hydrocarbon radical or an unsubstituted or substituted aliphatic or aromatic heterocyclic radical which is linked through a carbon atom to the CH group in formula I, by reacting a zinc compound with an aldehyde, which process comprises reacting an aldehyde of formula II

$$R-CHO \qquad (II)$$

in which R is as defined above, in an inert solvent, with a zinc compound of formula III

$$CF_3CCl_2ZnCl \cdot yL \qquad (III)$$

in which y is 1 or 2 and L is a solvent ligand selected from the group of the N-disubstituted acid amides, N-substituted lactams and organic sulfoxides, excepting the reaction of $CF_3CCl_3$ with an aldehyde RCHO in the presence of zinc, and subsequently isolating the 1-propanol of formula I.

2. A process according to claim 1, wherein the reaction is carried out at room temperature.

3. A process according to claim 1, wherein the reaction is carried out under the action of ultrasonics.

4. A process according to claim 1, wherein the solvent is a polar aprotic solvent.

5. A process according to claim 1, wherein the solvent is an N-substituted acid amide.

6. A process according to claim 1, wherein the 1-propanol of formula I is isolated by treating the reaction mixture with a dilute aqueous acid.

7. A process according to claim 1, wherein the inert solvent corresponds to the solvent ligand L in formula III.

8. A process according to claim 1, wherein equimolar amounts of the aldehyde of formula II and the zinc compound of formula III are used.

9. A process according to claim 1, wherein the zinc compound of formula III is prepared in a first reaction step in situ and then reacted with an aldehyde of formula II.

10. A process according to claim 1, wherein a zinc compound of formula III is dissolved in an inert solvent and then reacted with an aldehyde of formula II.

11. A process according to claim 1, wherein the reaction is carried out in an inert protective gas.

12. A process according to claim 1, wherein L in formula III is an N-disubstituted carboxamide.

13. A process according to claim 1, wherein y in formula III is 2.

14. A process according to claim 1, wherein the zinc compound of formula III is $CF_3CCl_2ZnCl \cdot 2$ dimethylformamide.

15. A process according to claim 1, wherein R in formula II as a hydrocarbon radical contains 1 to 30 carbon atoms and, as a heterocyclic radical, contains 1 to 3 heteroatoms in the ring.

16. A process according to claim 1, wherein substituents for R in formula II are: $C_1$–$C_{18}$alkyl, $C_2$–$C_{18}$alkenyl, $C_2$–$C_{18}$alkynyl, $C_6$–$C_{18}$aryl, $C_7$–$C_{18}$aralkyl, $C_7$–$C_{18}$alkaryl, $C_8$–$C_{18}$alkarylalkyl, $C_1$–$C_{18}$alkoxy, $C_1$–$C_{18}$alkylthio, $C_6$–$C_{18}$aryloxy, $C_7$–$C_{18}$aralkoxy, $C_7$–$C_{18}$alkaryloxy, $C_8$–$C_{18}$alkaralkoxy, $C_7$–$C_{18}$aryloxyalkyl, $C_7$–$C_{18}$alkoxyaryl, $C_3$–$C_{12}$cycloalkyl, $C_3$–$C_{12}$cycloalkoxy, $C_3$–$C_{12}$cycloalkenyl, $C_3$–$C_{12}$cycloalkenyloxy, $-COOH$, $-CONH_2$, $-COOR^1$, $-CONHR^1$, $-CONR^1R^2$, in which $R^1$ is $C_1$–$C_{18}$alkyl, cyclohexyl, phenyl or benzyl and $R^2$, independently has the same meaning as $R^1$; $-CN$, $-OH$, $-SH$, $-SO_2H$, $-SO_3H$, $-SO_2R^1$, $-SO_3R^1$, $-PO_2H$, $-PO_3H_2$, $-PO_2R^1$, $-PO_3R^1$, $-NO_2$, $-NH_2$, $-NHR^1$, $-NR^1R^2$, F, Cl, Br, I and $-NCO$ and the aliphatic radicals R may be interrupted by $-CO-$ or $-C(O)O-$.

17. A process according to claim 1, wherein R in formula II is monosubstituted to trisubstituted.

18. A process according to claim 1, wherein R in formula II, as an aliphatic hydrocarbon radical, is unsubstituted or substituted alkyl, alkenyl, alkynyl, cycloalkyl or cycloalkenyl, as an unsubstituted or substituted aromatic hydrocarbon radical is aryl, aralkenyl, aralkynyl or aralkyl, as an unsubstituted or substituted heterocyclic aliphatic radical, is a 4- to 6-membered heterocyclic ring which contains 1 or 2 heteroatoms and, as an aromatic heterocyclic radical, is a 5- or 6-membered heterocyclic ring which contains 1 or 2 heteroatoms.